# DEMANDE DE BREVET EUROPEEN

(11) **EP 0 968 708 A1**
(43) Date de publication de la demande: **05.01.2000**
(21) Numéro de dépôt: 99401296.1
(22) Date de dépôt: 31.05.1999
(51) Int. Cl.: A61K 7/48

(54) **Composition cosmétique anhydre, comprenant un silicone oxyalkyléné substitué en alpha-oméga et au moins un pigment**

(30) Priorité: 25.06.1998 FR 9808084
(71) Demandeur: L'OREAL, 75008 Paris (FR)
(72) Inventeur: Lemann, Patricia, 94000 Creteil (FR); Collette, Annick, 94600 Choisy le Roi (FR); Bara, Isabelle, 75013 Paris (FR)
(74) Mandataire: Brédeville, Odile Marie

(57) **Abrégé**

La présente invention a pour objet une composition anhydre stable, à usage cosmétique ou pharmaceutique, comprenant au moins une huile de silicone et des pigments, caractérisée par le fait qu'elle comprend en outre au moins une silicone oxyalkylénée substituée en α-ω.

## Description

La présente invention a pour objet des compositions cosmétiques anhydres à usage cosmétique ou pharmaceutique.

Les compositions de maquillage contenant une phase grasse sont couramment utilisées en cosmétique en raison de leur bonne adhérence à l'épiderme, leur sensation de confort, leur capacité protectrice et leur capacité à former un film imperméable à l'eau. Les produits anhydres de maquillage se présentent en général sous forme solide compacte ou bien sous forme de crème. Ils peuvent également se présenter sous la forme d'un gel fluide.

Ces compositions peuvent constituer des produits de soin de la peau, y compris le cuir chevelu, et/ou des produits de maquillage de la peau, des muqueuses (lèvres ou intérieurs des paupières), des semi-muqueuses (lèvres), des fibres kératiniques (cheveux, cils, ongles) ou encore des produits de maquillage du corps.

Toutefois, un des inconvénients de ce type de produit est qu'ils ne sont pas stables.

De plus, ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, un vêtement ou la peau.

En se déposant, ladite composition laisse une trace sur ledit support. Il s'en suit donc une persistance médiocre de la composition sur la peau ou les muqueuses, d'où la nécessité de renouveler régulièrement son application.

Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, générées par les mouvements des paupières.

Tous ces phénomènes engendrent un effet inesthétique que l'on souhaite bien évidemment éviter.

Dans le but de diminuer ces phénomènes, il a été proposé dans WO 97/16157 d'associer à un solvant volatil un tensio-actif polymérique de type organosiloxane ayant au moins un radical hydrophile et au moins un radical lipophile.

Toutefois, si l'on désire introduire des pigments dans ces produits, on se trouve souvent confronté à un problème d'homogénéité des pigments.

Dans tous les cas, les compositions obtenues présentent des défauts tant au niveau de la stabilité qu'au niveau de l'homogénéité de la dispersion des pigments.

Le but de la présente invention est de fournir une composition anhydre qui présente une bonne stabilité, qui transfère peu et qui présente une très bonne dispersion des pigments et ainsi des propriétés cosmétiques améliorées.

Il a maintenant été découvert de façon inattendue et surprenante que par l'emploi d'un tensio-actif particulier, il était possible d'obtenir des compositions anhydres ayant non seulement une bonne stabilité dans le temps mais encore vis-à-vis des variations de température et présentant de plus d'excellentes propriétés cosmétiques, en particulier une dispersion homogène des pigments.

La présente invention a donc pour objet une composition anhydre, en particulier à usage cosmétique, dermatologique, hygiénique ou pharmaceutique, comprenant au moins une huile de silicone et au moins un pigment, caractérisée par le fait qu'elle comprend au moins une silicone oxyalkylénée substituée en α-ω.

L'invention porte également sur un procédé de traitement non thérapeutique de la peau et/ou du cuir chevelu, notamment un procédé de maquillage, consistant à appliquer sur la peau ou les muqueuses et/ou sur le cuir chevelu une composition telle que définie ci-dessus.

La composition anhydre selon l'invention est particulièrement stable.

La composition anhydre selon l'invention est particulièrement homogène. Elle permet un maquillage uniforme et homogène.

La composition anhydre selon l'invention présente en outre une bonne résistance au transfert. De plus, appliquée sur la peau, elle présente l'avantage de ne pas migrer dans les plis de la peau et/ou les rides du visage.

On a constaté que la composition utilisée selon l'invention s'applique et s'étale facilement de façon homogène, sans laisser de sensation de gras et présente de bonnes propriétés cosmétiques. Le film obtenu présente également une texture légère et reste confortable à porter tout au long de la journée.

La composition selon l'invention possède par ailleurs de bonnes qualités sensorielles notamment une grande facilité d'application, du confort, de la douceur, une bonne matité et une bonne couvrance, de l'uniformité et de la tenue.

Les compositions de l'invention sont des compositions anhydres. Par composition anhydre, on entend une composition comprenant moins de 5 % en poids d'eau, par rapport au poids total de la composition, de préférence entre 1 % et 2 % d'eau. Plus préférentiellement encore, la composition ne comprend pas d'eau du tout. De préférence, les compositions de l'invention sont exemptes d'alcools polyvalents, c'est-à-dire d'alcools comprenant au moins deux groupes OH comme le propylène glycol, le butylène glycol, la glycérine, le sorbitol.

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

Ainsi, la silicone oxyalkylénée substituée en α-ω utilisable pour la composition selon l'invention est un polymère organosilicié tel que défini ci-dessus, à structure linéaire, substitué aux deux extrémités de la chaîne principale par des groupements oxyalkylène reliés aux atomes de Si par l'intermédiaire d'un groupement hydrocarboné. De préférence, la chaîne principale ne comprend pas de groupement oxyalkylène pendant.

De préférence, la silicone oxyalkylénée substituée en α-ω répond à la formule générale (I) suivante : 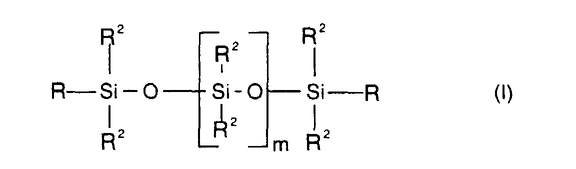 dans laquelle :

R = (CH₂)ₚ-O-(C₂H₄O)ₓ(C₃H₆O)_{Y}R¹

où :
- R¹ représente H, CH₃ ou CH₂CH₃,
- p est un entier allant de 1 à 5, x varie de 1 à 100, y varie de 0 à 50,
- les unités (C₂H₄O) et (C₃H₆O) pouvant être réparties de façon aléatoire ou par blocs,
- les radicaux R² représentent un radical alkyle en C1-C3 ou un radical phényle,
- 5 ≤ m ≤ 300.

De préférence, la silicone oxyalkylénée substituée en α-ω utilisée selon la présente invention répond à la formule générale (I) pour laquelle tous les radicaux R² sont des radicaux méthyles et :
- p va de 2 à 4,
- x va de 3 à 100,
- m va de 50 à 200.

De préférence encore, le poids moléculaire moyen de R va de 800 à 2600.

De préférence, le rapport en poids des unités C₂H₄O par rapport aux unités C₃H₆O va de 100:10 à 20:80.

De préférence, ce rapport est d'environ 42/58.

De préférence encore, R¹ est le groupe méthyle.

De façon plus préférentielle encore, la composition selon l'invention comprend la silicone oxyalkylénée substituée en α-ω de formule suivante : 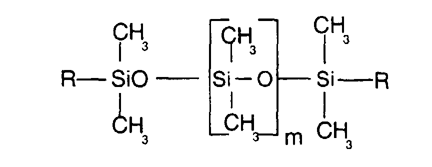 dans laquelle :
- m = 100,
- R = (CH₂)₃-O-(C₂H₄O)ₓ (C₃H₆O)_{y}-CH₃, où x va de 3 à 100, y va de 1 à 50, le rapport en poids du nombre de C₂H₄O sur le nombre de C3H6O étant d'environ 42/58, le poids moléculaire moyen de R allant de 800 à 1500.

La silicone oxyalkylénée substituée en α-ω telle que définie ci-dessus est utilisée selon l'invention en une proportion allant de 0,1 à 20 % de préférence allant de 0,1 à 10 % en poids par rapport au poids total de la composition.

Parmi les produits du commerce pouvant contenir tout ou partie des silicones oxyalkylénées substituées en α-ω utilisables selon l'invention comme émulsionnant on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.

Les compositions selon l'invention comprennent au moins un pigment.

Les pigments peuvent être présents dans la composition à une teneur allant de 0,1 à 20 % en poids, par rapport au poids total de la composition, et de préférence à raison de 2-15 %. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré. Parmi les pigments organiques, on peut citer le noir de carbone, et les laques de baryum, strontium, calcium, aluminium. Les pigments peuvent également présenter une surface hydrophobe ou peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères, notamment des polyéthylènes et/ou des acides aminés.

Parmi les pigments enrobés, on peut citer notamment les pigments vendus sous la dénomination de "Covasil" par la Société WACKER (pigments au triisostéaroyltitanate).

Les pigments ainsi enrobés peuvent être incorporés dans la composition selon l'invention en une proportion comprise entre 0,1 et 15 % en poids par rapport au poids total de la composition.

Les compositions selon l'invention comprennent au moins une huile de silicone volatile ou non.

L'huile de silicone utilisable selon l'invention peut être un polydiorganosiloxane linéaire, éventuellement fonctionnalisé, ou cyclique ou un organopolysiloxane éventuellement réticulé ou un mélange de ceux-ci.

Les polydiorganosiloxanes linéaires éventuellement fonctionnalisés utilisables selon l'invention répondent à la formule générale suivante : 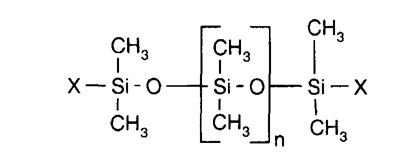 dans laquelle :
- X est -CH3 ou OH, et
- n est un entier allant de 0 à 2000.

Parmi ceux-ci, on citera notamment les produits vendus sous la dénomination de "AK" par la Société WACKER, "SF" par la Société GENERAL ELECTRIC et "ABIL" par la Société GOLDSCHMIDT, tel que le produit "Abil 10".

Comme polydiorganosiloxanes cycliques selon l'invention, on peut utiliser, seuls ou en mélange, des cyclométhicones de formule : 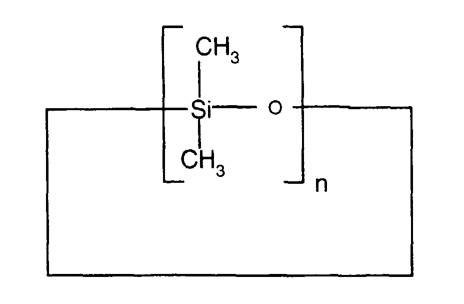 dans laquelle :
- n est un nombre entier de 3 à 8.

Parmi les cyclométhicones particulièrement préférées, on citera le cyclotétradiméthylsiloxane (n = 4), le cyclopentadiméthylsiloxane (n = 5), et le cyclohexadiméthylsiloxane (n = 6).

On peut notamment utiliser les produits vendus sous les dénominations de "DC Fluid 244", "DC Fluid 245", "DC FLuid 344" et "DC Fluid 345" par la Société DOW CORNlNG.

D'autres cyclométhicones utilisables selon l'invention sont celles vendues sous les dénominations de "Abil K4" par la Société GOLDSCHMIDT ; sous les dénominations de "Silbione 70045 V2" et de "Silbione Huile 70045 V5" par la Société RHONE POULENC ; ainsi que sous les dénominations de "Volatil Silicone 7158" et de "Volatil Silicone 7207" par la Société UNION CARBIDE.

De préférence, on utilise les huiles de silicone volatiles, et de préférence encore les cyclométhicone.

Tel qu'indiqué ci-dessus, l'huile de silicone utilisée selon l'invention est de préférence présente en une proportion d'au moins 5 % et de préférence allant de 5 à 80 % en poids par rapport au poids total de la composition.

Les compositions de l'invention peuvent également comprendre des alkyl, alcoxy ou phényl-diméthicones tels que, par exemple le produit vendu sous la dénomination de "Abil wax 2440" par la Société GOLDSCHMIDT.

Les compositions selon l'invention peuvent également comprendre des résines de silicone comprenant une combinaison des unités R₃SiO_{1/2}, R₂SiO_{2/2}, RSiO_{3/2} et SiO_{4/2}, R représentant un groupe alkyle ayant de 1 à 6 atomes de carbone ou un groupe phényle.

Les compositions selon l'invention peuvent également comprendre d'autres corps gras siliconés.

Parmi les corps gras siliconés, on peut citer les polyalkyl(C₁-C₂₀)siloxanes, les huiles de silicone phénylées, ainsi que les gommes de silicones et les cires de silicone.

Les gommes de silicone peuvent répondre à la formule : 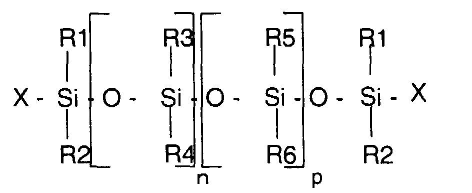 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle et notamment phényle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.
De manière générale, n et p peuvent prendre des valeurs de 0 à 5000, de préférence de 0 à 3000.

Comme gomme de silicone utilisable selon l'invention, on peut citer celles pour lesquelles :
- les substituants R1 à R6 et X représentent un groupement méthyle, p = 0 et n = 2700, comme celle vendue sous la dénomination SE30 par la société General Electric,
- les substituants R1 à R6 et X représentent un groupement méthyle, p = 0 et n = 2300, comme celle vendue sous la dénomination AK 500000 par la société Wacker,
- les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
- les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
- les substituants R1, R2, R5, R6 et X représentent un groupement méthyle, les substituants R3 et R4 représentent un groupement phényle tel que le poids moléculaire du composé soit de 600 000, comme celle vendue sous les dénominations "761" ou "MIRASIL C-DPDM" par la société Rhône-Poulenc.

La composition selon l'invention peut comprendre en outre des corps gras non siliconés.

Parmi les corps gras non siliconés, on peut citer l'huile de paraffine, de vaseline, le perhydrosqualène, l'huile d'abricot, l'huile de germes de blé, d'amande douce, de calophyllum, de palme, de ricin, d'avocat, de jojoba, d'olive, l'huile de tournesol, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de noyau; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C; la cire d'abeilles; les cires végétales telles que la cire de Carnauba, de Candellila, d'Ouricury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

Ces corps gras peuvent en particulier être choisis de manière variée par l'homme du métier afin de préparer une composition ayant les propriétés souhaitées, par exemple en consistance ou en texture.

Parmi les autres adjuvants liposolubles que l'on peut incorporer à la composition, on peut citer les filtres U.V. lipophiles, les vitamines lipophiles, les antioxydants et les parfums, les céramides.

Les compositions selon l'invention peuvent également comprendre une phase particulaire qui peut comprendre, outre les pigments cités ci-dessus, des nacres et/ou des charges habituellement utilisés dans les compositions cosmétiques.

Les charges, qui peuvent être présentes dans la composition à raison de 0-20 % en poids, par rapport au poids total de la composition, de préférence 0-10 %, peuvent être minérales ou de synthèse, lamellaires ou non lamellaires. On peut citer le talc, le mica, la silice, le kaolin, le Téflon, l'amidon, la nacre naturelle, le nitrure de bore, les microsphères telles que l'Expancel (Nobel Industrie), les microéponges telles que le polytrap (Dow Corning). De préférence, on utilise des charges sphériques dont la taille est inférieure à 25 µm telles que les poudres de polyéthylène, les poudres de Nylon, les microbilles de résine de silicone (Tospearls de Toshiba), les microsphères de silice, ces charges pouvant contribuer à améliorer les propriétés non transfert des compositions de l'invention.

La composition selon l'invention peut comprendre en outre un milieu cosmétiquement, pharmaceutiquement ou hygiéniquement acceptable. Elle peut comprendre alors tout additif usuellement utilisé dans le domaine cosmétique,
pharmaceutique ou hygiénique, tels que des antioxydants, des colorants, des parfums, des huiles essentielles, des conservateurs, des actifs cosmétiques, des hydratants, des vitamines, des sphingolipides, des polymères liposolubles notamment hydrocarbonés, tels que le polybutène, les polyalkylènes, les polyacrylates et les polymères siliconés compatibles avec les corps gras.

Ces additifs peuvent être présents dans la composition à raison de 0-10% en poids.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme d'un produit cosmétique et notamment sous la forme d'un produit de soin pour le corps et/ou le visage et/ou le cuir chevelu ou bien encore d'un produit de maquillage, en particulier un fond de teint, un fard à joues ou à paupières, un eye-liner, un mascara ou un rouge à lèvres.

Elles peuvent également se présenter sous forme non colorée, contenant éventuellement des actifs cosmétiques.

Les compositions selon l'invention peuvent se présenter sous la forme d'un gel fluide ou d'un stick.

De préférence, les compositions selon l'invention se présentent sous une forme fluide et ont une viscosité Brookfield, mesurée sur le modèle LVDV, avec une aiguille de 3, à une vitesse de 60 trs/min et à environ 25°C, allant de 8 poises à 50 poises.

L'invention porte encore sur l'utilisation d'une silicone oxyalkylénée substituée en α-ω telle que définie ci-dessus dans une composition anhydre comprenant des pigments et une huile de silicone dans le but d'améliorer la dispersion desdits pigments dans ladite composition.

L'invention porte également sur l'utilisation d'une silicone oxyalkylénée substituée en α-ω telle que définie ci-dessus dans une composition anhydre comprenant des pigments et une huile de silicone dans le but d'améliorer l'homogénéité de ladite composition.

### EXEMPLE COMPARATIF :

La Demanderesse a réalisé les deux compositions anhydres suivantes :

### Composition A (conforme à l'invention) :

- cyclométhicone 97,5 g
- mélange silicone oxyéthylénée oxypropylénée substituée en α-ω /cyclométhicone (85/15) vendu sous la dénomination commerciale "Abil EM 97" par la Société Goldschmidt 2,5 g
- pigments 50 g

### Composition B (comparative) :

- cyclométhicone 97,5 g
- silicone à groupements alkyle, oxyéthylène et oxypropylène pendants dans un mélange polyglycéryl-4-isostéarate et hexyllaurate vendue sous la dénomination commerciale "Abil WE 09" par la société Goldschmidt 2,5 g
- pigments 50 g

La Demanderesse a ensuite mesuré les viscosités au temps t = 3 minutes des compositions A et B. Ces viscosités ont été mesurées au Brookfield, modèle LVDV, avec une aiguille de 3, à une vitesse de 60 trs/min et à environ 25°C. Les résultats sont regroupés dans le tableau suivant:

| **Composition** | **Viscosité Brookfield** en poises |
|---|---|
| A (conforme à l'invention) | 8,8 |
| B (comparative) | 14 |

Ainsi, la composition anhydre A, pour le même taux de pigment, est beaucoup plus fluide que la composition B. La composition A présente une texture plus homogène que la composition B. Les pigments sont mieux dispersés dans la composition A selon l'invention que dans la composition B.

## Revendications

1. Composition anhydre comprenant au moins une huile de silicone et au moins un pigment, caractérisée par le fait qu'elle comprend au moins une silicone oxyalkylénée substituée en α-ω.

2. Composition selon la revendication 1, caractérisée par le fait que la silicone oxyalkylénée substituée en α-ω est un polymère organosilicié à structure linéaire, substitué aux deux extrémités de la chaîne principale par des groupements oxyalkylène reliés aux atomes de Si par l'intermédiaire d'un groupement hydrocarboné.

3. Composition selon la revendication 2, caractérisée par le fait que la chaîne principale ne comprend pas de groupement oxyalkylène pendant.

4. Composition selon l'une quelconque des revendications 1 à 3, caractérisée par le fait que la silicone oxyalkylénée substituée en α-ω répond à la formule générale (I) suivante : 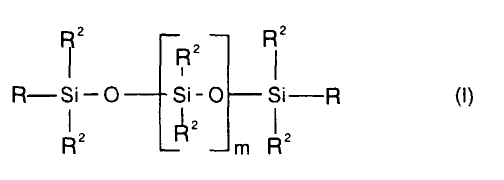 dans laquelle :
R = -(CH₂)ₚ-O-(C₂H₄O)ₓ (C₃H₆O)_{Y}R¹
où :
- R¹ représente H, CH₃ ou CH₂CH₃,
- p est un entier allant de 1 à 5, x varie de 1 à 100, y varie de 0 à 50,
- les unités (C₂H₄O) et (C₃H₆O) pouvant être réparties de façon aléatoire ou par blocs,
- les radicaux R² représentent un radical alkyle en C1-C3 ou un radical phényle,
- 5 ≤ m ≤ 300.

5. Composition selon la revendication 4, caractérisée par le fait que la silicone oxyalkylénée substituée en α-ω utilisée selon la présente invention répond à la formule générale (I) pour laquelle tous les radicaux R² sont des radicaux méthyles et :
- p va de 2 à 4,
- x va de 3 à 100,
- m va de 50 à 200.

6. Composition selon la revendication 4 ou 5, caractérisée par le fait que le poids moléculaire moyen de R va de 800 à 2600.

7. Composition selon l'une quelconque des revendications 4 à 6, caractérisée par le fait que le rapport en poids des unités C₂H₄O par rapport aux unités C3H6O va de 100:10 à 20:80.

8. Composition selon la revendication 7, caractérisée par le fait que ce rapport est d'environ 42/58.

9. Composition selon l'une quelconque des revendications 4 à 8, caractérisée par le fait qu'elle comprend la silicone oxyalkylénée substituée en α-ω de formule suivante : 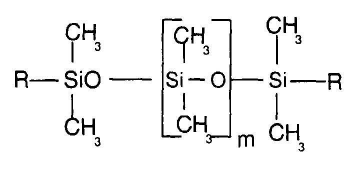 dans laquelle :
- m = 100,
- R = (CH₂)₃-O-(C₂H₄O)ₓ (C₃H₆O)_{y}-CH₃, où x va de 3 à 100, y va de 1 à 50, le rapport en poids du nombre de C₂H₄O sur le nombre de C₃H₆O étant d'environ 42/58, le poids moléculaire moyen de R allant de 800 à 1500.

10. Composition selon l'une quelconque des revendications 1 à 9, caractérisée par le fait que la silicone oxyalkylénée substituée en α-ω est présente dans la composition en une proportion allant de 0,1 à 20 % en poids par rapport au poids total de la composition

11. Composition selon la revendication 10, caractérisée par le fait que cette proportion va de 0,1 à 10 % en poids par rapport au poids total de la composition.

12. Composition selon l'une quelconque des revendications 1 à 11, caractérisée par le fait que les pigments sont choisis parmi les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré, le noir de carbone, les laques de baryum, strontium, calcium, aluminium, les pigments enrobés par des composés siliconés tels que des PDMS et/ou par des polymères, notamment des polyéthylènes et/ou des acides aminés.

13. Composition selon l'une quelconque des revendications 1 à 12, caractérisée par le fait que les pigments sont présents dans la composition à une teneur allant de 0,1 à 20% en poids, par rapport au poids total de la composition.

14. Composition selon la revendication 13, caractérisée par le fait que cette teneur va de 2 à 15% en poids, par rapport au poids total de la composition.

15. Composition selon l'une quelconque des revendications 1 à 14, caractérisée par le fait que l'huile de silicone est choisie parmi les polydiorganosiloxanes linéaires, éventuellement fonctionnalisés, ou cycliques ou les organopolysiloxanes éventuellement réticulés ou un mélange de ceux-ci.

16. Composition selon la revendication 15, caractérisée par le fait que l'huile de silicone est un polydiorganosiloxane cyclique.

17. Composition selon l'une quelconque des revendications 1 à 16, caractérisée par le fait que l'huile de silicone est présente dans la composition en une proportion d'au moins 5 % en poids par rapport au poids total de la composition.

18. Composition selon la revendication 17, caractérisée par le fait que cette teneur va de 5% à 80% en poids, par rapport au poids total de la composition.

19. Composition selon l'une quelconque des revendications 1 à 18, caractérisée par le fait qu'elle comprend en outre un corps gras choisi parmi l'huile de paraffine, de vaseline, le perhydrosqualène, l'huile d'abricot, l'huile de germes de blé, d'amande douce, de calophyllum, de palme, de ricin, d'avocat, de jojoba, d'olive, l'huile de tournesol, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de noyau; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C; la cire d'abeilles; les cires végétales telles que la cire de Carnauba, de Candellila, d'Ouricury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

20. Composition selon l'une quelconque des revendications 1 à 19, caractérisée par le fait qu'elle comprend en outre une charge choisie parmi le talc, le mica, la silice, le kaolin, le Téflon, l'amidon, la nacre naturelle, le nitrure de bore, les microsphères, les microéponges, les poudres de polyéthylène, les poudres de Nylon, les microbilles de résine de silicone, les microsphères de silice.

21. Composition selon l'une quelconque des revendications 1 à 20, caractérisée par le fait qu'elle est à usage cosmétique, dermatologique, hygiénique ou pharmaceutique.

22. Composition selon l'une quelconque des revendications 1 à 21, caractérisée par le fait qu'elle se présente sous la forme d'un produit de soin pour le corps et/ou le visage et/ou le cuir chevelu ou bien encore d'un produit de maquillage, en particulier un fond de teint, un fard à joues ou à paupières, un eye-liner, un mascara ou un rouge à lèvres.

23. Composition selon l'une quelconque des revendications 1 à 22, caractérisée par le fait qu'elle se présente sous la forme d'un gel fluide ou d'un stick.

24. Procédé de traitement non thérapeutique de la peau et/ou du cuir chevelu, notamment un procédé de maquillage, consistant à appliquer sur la peau ou les muqueuses et/ou sur le cuir chevelu une composition telle que définie à l'une quelconque des revendications 1 à 23.

25. Utilisation d'une silicone oxyalkylénée substituée en α-ω telle que définie à l'une quelconque des revendications 1 à 9 dans une composition anhydre comprenant des pigments et une huile de silicone dans le but d'améliorer la dispersion desdits pigments dans ladite composition.

26. Utilisation d'une silicone oxyalkylénée substituée en α-ω telle que définie à l'une quelconque des revendications 1 à 9 dans une composition anhydre comprenant des pigments et une huile de silicone dans le but d'améliorer l'homogénéité de ladite composition.
